# EUROPEAN PATENT APPLICATION

(11) **EP 1 380 273 A2**
(43) Date of publication of application: **14.01.2004**
(21) Application number: 03253489.3
(22) Date of filing: 03.06.2003
(51) Int. Cl.: A61F 2/38

(54) **Prosthetic knee system**

(30) Priority: 04.06.2002 US 161754
(71) Applicant: Zimmer Technology, Inc., Chicago, Illinois 60606 (US)
(72) Inventor: Donkers, Ronald G., Warsaw, IN 46580 (US); Perry, Alyssa M., Fort Wayne, IN 46804 (US); Steffensmeier, Scott J., Warsaw, IN 46580 (US); Tanamal, Linggawati, Fort Wayne, IN 46804 (US); Zawakzki, Michelle, Leesburg, IN 46538 (US)
(74) Representative: Mays, Julie

(57) **Abstract**

A femoral component and provisional femoral component system for use in an endoprosthetic knee joint. The femoral component system of the present invention comprises a universal contacting surface geometry and a plurality of unique external articulating surface geometries to accommodate a variety of knee joint sizes and knee ligament tension situations.

## Description

### BACKGROUND

The present invention relates to a provisional and final femoral component for an endoprosthetic knee.

### DESCRIPTION OF THE RELATED ART

Endoprosthetic knees of various designs are well known in orthopaedics. Such devices have often been used to successfully treat knee injuries and ailments.

Generally, endoprosthetic knees comprise a femoral component, a tibial component and a meniscal component, sometimes referred to as an articular bearing surface, disposed there between. The femoral component comprises an internal bone contacting surface and an external articulating surface. The internal surface comprises a specific desired shape.

During a total knee arthroplasty ("TKA") or partial knee arthroplasty ("PKA"), a surgeon cuts the distal femur of the patient into a shape corresponding to the internal surface of the femoral component. The surgeon also resects the proximal tibia, then chooses and attached a desired tibial component. The surgeon subsequently chooses a provisional femoral component and provisional nenixal components. After attaching the provisional femoral component and inserting the provisional meniscal component, the surgeon "reduces" the knee joint to determine whether the proper sizes of components have been selected. If the patient's joint is unable to accommodate a desirable range of motion due to the size of the implants or if there is too much laxity in the joint due to the same, the surgeon will remove the provisional implants and replace them with differently sized provisional implants.

If there is a need to change the size of the provisional femoral component, the surgeon must often make new cuts to the distal femur. If necessary, the surgeon may need to repeat this process until he or she is satisfied with the fit of the particular endoprosthetic knee, and can replace the provisional components with final components.

It is desirable, therefore, to provide an endoprosthetic knee implant system that alleviates the need for the surgeon to recut bone in order to achieve the proper fit for a femoral knee implant component.

### SUMMARY OF THE INVENTION

It is an object of the present invention, therefore, to provide an endoprosthetic knee implant system wherein the components are designed such that the surgeon does not need to cut additional bone from a patient's distal femur to change the size of a provisional femoral component.

The present invention comprises a set of provisional and final femoral knee implant components, wherein each component comprises a unique external articulating surface geometry, but wherein the components have a constant internal bone contacting surface geometry.

Thus, an advantage of the present invention is that a surgeon performing a total or partial knee arthroplasty who finds that the motion of the patient's knee is impinged or that the knee is too lax throughout a desired range of motion, may simply remove one provisional femoral implant and replace it with another implant having the same internal surface geometry but a slightly smaller external or larger external geometry without making new cuts on the patient's distal femur.

Although the present invention is described herein as providing a single internal geometry for a pair of external geometries comprising different anterior to posterior dimensions, those skilled in the art of orthopaedic implants will understand that a single internal geometry could also be used to accommodate any number of implants having unique medial to lateral, inferior to superior, or anterior to posterior external dimensions, and a constant internal geometry.

### BRIEF DESCRIPTION OF THE DRAWINGS

FIG. 1 is a side elevational of an endoprosthetic knee according to the present invention.
FIG. 2 is a perspective view of a femoral component of an endoprosthetic knee according to the present invention.
FIG. 3 is a perspective view of a pair of artificial femoral components according to the present invention.
FIG. 4 is a perspective view of a provisional femoral component according to another embodiment of the present invention.

### DETAILED DESCRIPTION OF THE DRAWINGS

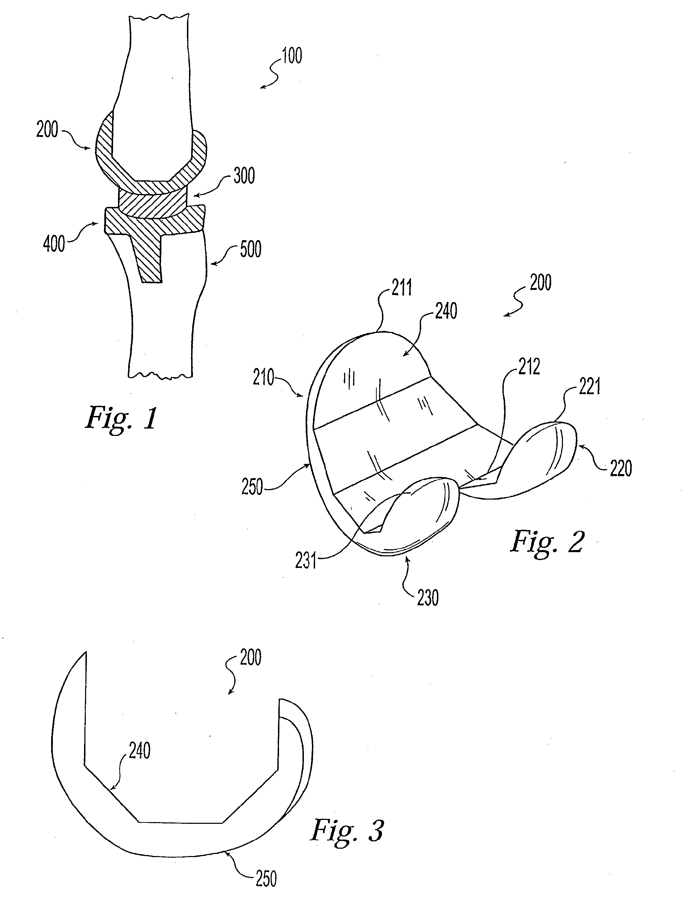

The present invention relates to the femoral component of an endoprosthetic knee. Referring now to FIG.1, an endoprosthetic knee comprises femoral component 200, tibial plate 400 and articulating bearing surface 300 disposed there between. Prosthetic knee joints are generally required where all, or a portion of a patient's natural knee has become painful, misaligned or dis-functional due to trauma or disease. Where a surgeon replaces an entire knee joint (ie: both femoral condyles, the entire meniscus, and the natural proximal tibia), the surgical procedure is referred to as a total knee arthroplasty. Where only one condyle is replaced the procedure is referred to as a partial knee arthroplasty. For the sake of clarity, the present invention is described in terms of a total knee arthroplasty. However, those skilled in the art will appreciate that the invention is equally applicable to a partial knee arthroplasty.

Referring still to FIG. 1, there is shown femur 100 having a series of cuts made to its distal end (a resected femur). Although a specific shape of femoral cuts is shown and described herein, those skilled in the art will appreciate that any series of cuts may be made to the distal femur and any matching internal femoral component geometry may be used within the scope of the present invention. During a total knee arthroplasty this series of cuts, commonly referred to as "box cuts," is made to the distal femur in order to remove diseased or damaged bone and to prepare the distal femur to receive femoral component 200. Similarly, a generally flat cut is made to proximal tibia 500 to prepare the tibia 500 to receive tibial plate 400. The box cuts made to distal femur 100 are adapted to allow femoral component 200 to be fixedly attached thereto. The fixed attachment may be accomplished by any, or a combination of, several means known to those of skill in the art, such as using bone cement, using threaded bone screws, or using a porous surface that allows bone ingrowth to secure distal femur 100 and femoral component 200.

One issue with which physicians must contend is accommodating the size of an endoprosthetic knee in the amount of space available in a particular patient's knee joint cavity. Soft tissues, including skin, ligaments, tendons and the like operate to restrict the space available to an endoprosthetic joint

Presently, when a physician must change a femoral component to a larger or smaller size, new box cuts must be made to distal femur 100. The present invention comprises a system or set of femoral components and provisional components having a universal box cut geometry suitable for an infinite array of femoral component sizes, while prior devices and methods have required measuring the size of a distal femur 100 and cutting the bone to fit a specific size of prosthesis. Surgeons also sometimes try more than one size of provisional femoral component 200 to achieve an optimal range of motion between knee flexion and extension for a patient's knee joint given the soft tissue spacing issues described herein. Upon finding a provisional component of appropriate size, the physician chooses a final implant of equal size to use in the actual joint replacement. However, if a physician tries a first provisional femoral implant 200 according to the present invention and finds that it is too large or too small to operate properly in a patient's knee joint, a new provisional or final femoral component may be used in its place without the need to make additional cuts to distal femur 100.

Referring now to FIG.2, femoral component 200 comprises: anterior flange 210; medial and lateral condylar flanges 220 and 230; interior femoral bone contacting surface 240 and exterior articulating surface 250.

Anterior flange 210 comprises superior and inferior ends 211 and 212. Extending posteriorly from, and fixedly attached to, inferior end 212 of anterior flange 210 are parallel medial and lateral condylar flanges 220 and 230.

The condylar flanges comprise anterior ends 221 and 231 respectively which anterior ends 221 and 231 are fixedly attached to interior end 212 of anterior flange 210.

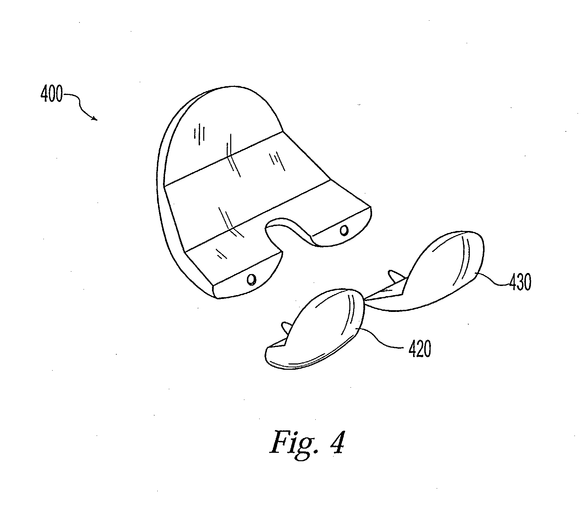

Referring to FIG. 2 and FIG. 3, each flange 210, 220 and 230 comprises a portion of interior bone contacting surface 240. Interior 240 comprises a series of connected, segmented planes adapted to be attachable to resected distal femur. The number of connected planes may be as many or as few as desired. Similarly any desired geometry of said planes is acceptable.

In an alternative embodiment of the present invention, condylar flanges 420 and 430 are removably attached to provisional femoral component 400. Such a modular configuration allows a surgeon to alter the size of femoral component 400 without removing the entire provisional component.

Referring again to FIG. 2, femoral component 200 further comprises exterior articulating surface 250. Articulating surface 250 is generally convex and comprises the exterior of each flange 210, 220 and 230. Anterior flange 210 preferably comprises patella tracking groove (not shown) disposed on articulating surface 250 along the longitudinal axis of anterior flange 210.

Femoral component 200 preferably comprises a bio compatible metal. A cobalt chrome alloy is preferred, but any rigid, resilient bio compatible material is acceptable.

The planes comprising interior 240 of femoral component 200 comprise a desired geometry matching the cuts made by the surgeon to distal femur 100. The geometric relationship between the planes remains relatively constant over an array of implant sizes.

Referring now to FIG. 3, there is shown a set of femoral implants 200 according to the present invention. Therein, interior bone contacting surface 240 comprises a series of interconnected planes having a geometric relationship to one another that is unchanged even as the dimensions or shape of articulating surface 250 are changed to accommodate soft tissues in the knee.

It will be appreciated by these skilled in the art that the foregoing is a description of the preferred embodiment of the present invention. Variations in design and construction may be made to the preferred embodiment without departing from the scope of the invention as defined by the appended claims.

## Claims

1. A set of femoral components for an endoprosthetic knee comprising at least two femoral components, wherein each of the at least two femoral components comprise an exterior articulating bearing surface; an interior bone contacting surface disposed opposite the articulating surface; an anterior flange; a medial posterior flange; and a lateral posterior flange.

2. The set of femoral components of Claim 1, wherein each of the at least two femoral components has a unique exterior inferior to superior height.

3. The set of femoral components of Claim 1, wherein each of the at least two femoral components has a unique exterior anterior to posterior length.

4. The set of femoral components of Claim 1, wherein each of the at least two femoral components has a unique exterior medial to lateral length.

5. The set of femoral components of claim 1,2,3 or 4, wherein the set of femoral components comprises provisional femoral components.

6. The set of femoral components of claim 5, wherein the medial and lateral posterior flanges are removably attached to the femoral components.
